(19) Europäisches Patentamt
European Patent Office
Office européen des brevets

(11) Publication number: 0 195 116 B1

(12) **EUROPEAN PATENT SPECIFICATION**

(45) Date of publication of patent specification: 17.11.88

(51) Int. Cl.$^4$: C 07 D 233/60, A 61 K 31/415

(21) Application Number: 85111157.5

(22) Date of filing: 04.09.85

(54) **Compound having antibacterial and antimycotic activities, preparation thereof and pharmaceutical compositions containing it.**

(30) Priority: 19.03.85 IT 1995785

(43) Date of publication of application: 24.09.86 Bulletin 86/39

(45) Publication of the grant of the patent: 17.11.88 Bulletin 88/46

(84) Designated Contracting States: AT BE CH DE FR GB IT LI LU NL SE

(56) References cited:
EP-A-0 000 017
FR-A-2 309 223
GB-A-2 025 395

CHEMICAL ABSTRACTS, vol. 105, no. 19, 10th November 1986, page 748, abstract no. 172456f, Columbus, Ohio, US; & BE-A-903 746 (PULITZER ITALIANA S.p.A.) 14-03-1986

(73) Proprietor: ISTITUTO FARMACO BIOLOGICO RIPARI GERO S.r.l.
Via Chiantigiana, 84
I-53035 Montearioso-Monteriggioni (Siena) (IT)

(72) Inventor: Ferrari, Giuseppe
Via Chiantigiana, 84
I-53035 Montearioso-Monteriggioni Siena (IT)

(74) Representative: Bianchetti, Giuseppe
Studio Consulenza Brevettuale Via Rossini, 8
I-20122 Milan (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 195 116 B1

**Description**

The present invention concerns the compound 1-{[2-(2,4-dichlorophenyl)-2-(2,4-dichlorophenyl)-methoxy]ethyl}-1-pivaloyloxymethyl-1H-imidazolium chloride, having formula I

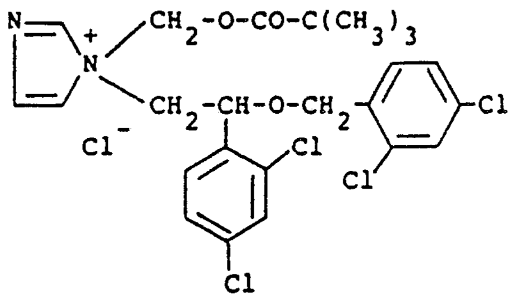

(I)

Compound I is endowed with remarkable antibacterial and antimycotic activities, and it is therefore useful in therapy. Another object of the invention is therefore provided by pharmaceutical compositions containing it as the active principle.

Compound I is prepared according to the invention by reacting 1-(pivaloyloxymethyl)imidazole, having formula II, with 1-chloro-2-(2,4-dichlorophenyl)-2-[(2,4-dichlorobenzyl)oxy]ethane, of formula III, according to the following scheme I:

SCHEME I

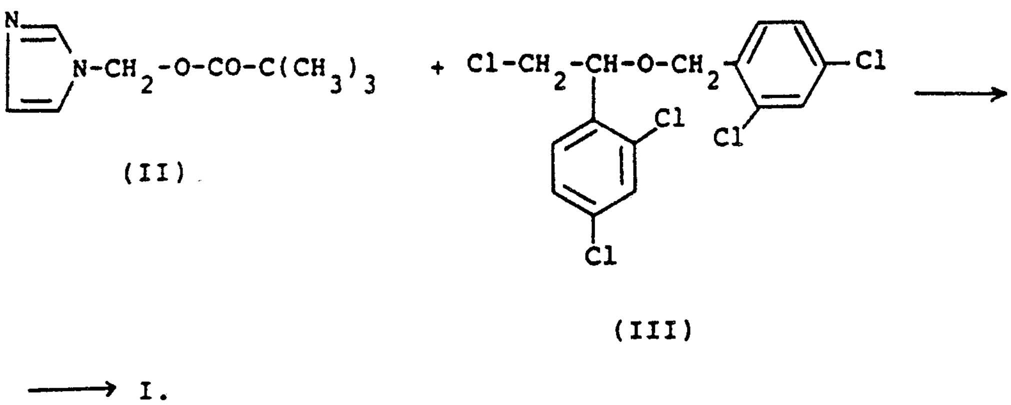

(II)

(III)

I.

Alternatively, compound I may be prepared by reaction of 1-[2,4-dichloro-β-[(2,4-dichlorobenzyl)oxy]-phenetyl]-imidazole, of formula IV, with pivalic acid chloromethylester, having formula V according to the following scheme II

SCHEME II

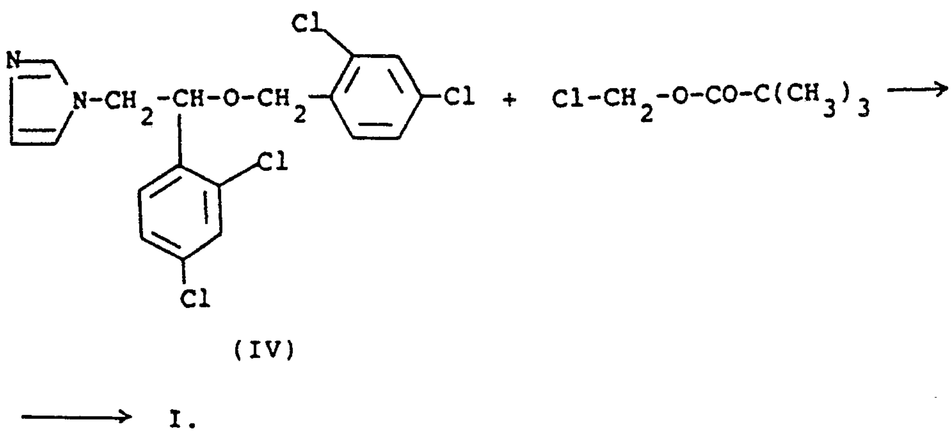

(IV)

I.

The following examples are given by way of illustrating the invention.

Example 1

1-{[2-(2,4-Dichlorophenyl)-2-(2,4-dichlorophenyl)methoxy]ethyl}-1-pivaloyloxymethyl-1H-imidazolium chloride, from 1-(pivaloyloxymethyl)imidazole and 1-chloro-2-(2,4-dichlorophenyl)-2-[(2,4-dichlorobenzyl)-oxy]ethane

An intimate mixture of 1-chloro-2-(2,4-dichlorophenyl)-2-[(2,4-dichlorobenzyl)oxy]ethane (3.84 g; 10 mmoles) and of 1-(pivaloyloxymethyl)imidazole (1.82 g; 10 mmoles) is heated in water bath under stirring to about 60°C for 2 hours.

After cooling, a glassy bulk, which is crystallized from a diethyl ether-ethanol (1:1) mixture, is obtained.

The analitically pure compound is obtained (4.1 g; 7.23 mmoles; yield 72.3%), having the following characteristics:

*Aspect:* white, microcrystalline slightly hygroscopic powder.

*Melting point:* 85°C (not correct — melting starts at 77°C).

*Solubility:* soluble in water, methanol and ethanol.

*Elemental analysis:* for $C_{24}H_{25}Cl_5N_2O_3$ (566.7)

| | C | H | N |
|---|---|---|---|
| Calc. % | 50.86 | 4.44 | 4.94 |
| Found % | 50.81 | 4.47 | 4.96. |

*Ionic chlorine* ($AgNO_3$): calc.% 6.25; found %: 6.30.

*I.R. Spectrum:* 1120 (—C—O—); 1745 (—C=O ester).

*$^1$H-NMR Spectrum:* (in $CDCl_3$; inn. st. TMS, chemical shifts in δ): 1.2 (s, 9H; —C($CH_3$)$_3$);

4.4 (s, 2H; $CH_2$—O—CH—);

4.7 (d, 2H; N—$CH_2$—CH—);

5.2 (t, 1H; —*CH*—O—);

6.2 (s, 2H; $\overset{+}{N}$—$CH_2$—O—CO—);

7.1—7.5 (m, 6H, aromatics).

Example 2

1-{[2-(2,4-Dichlorophenyl)-2-(2,4-dichlorophenyl)methoxy]ethyl}-1-pivaloyloxymethyl-1H-imidazolium chloride, from 1-[2,4-dichloro-β-[(2,4-dichlorobenzyl)oxy]phenethyl]imidazole and pivalic acid chloromethylester

The process described in Example 1 was repeated, using 1-[2,4-dichloro-β-[(2,4-dichlorobenzyl)oxy]-phenetyl]-imidazole (3.8 g; 9.13 mmoles) and pivalic acid chloromethylester (1.37 g; 9.13 mmoles) as reactants, to obtain a compound having the same chemical and chemico-physical characteristics as that obtained in Example 1.

Compound I, which will be hereinafter called also RG 024, for sake of shortness, displays remarkable antimycotic and antibacterial activities, higher or comparable to that of miconazole, being effective against yeasts and molds as well as gram-positive and gram-negative bacteria.

The microbiological tests carried out, hereinafter reported, further elucidate the activity of compound I.

*"In vitro" antimycotic activity*

The "in vitro" antimycotic activity of RG 024, in comparison with that of miconazole (M.I.C., i.e. minimum inhibiting concentration, expressed in mcg/ml) was evaluated in liquid culture medium (Get Nitrogen Base DIFCO with 5% of glucose) against myceti from hospital isolation wards.

The method is that of scalar dilutions of the test compounds on Microtiter plates, in a 1:2 dilution ratio. Suspensions (titre: $10^4$) of fungal sporae and Candida cells, grown on Sabouraud, and Dextrose Agar, were collected at the moment of use in the same medium used for the test and inoculated by means of a multiinoculation Microtiter device.

M.I.C. values were determined by means of visual observation of fungal growth respectively after 24 hours (Candida albicans) and 48 and 72 hours (dermatophytes) incubation at 24°C.

The results are shown in Table 1.

TABLE 1
"In vitro" antimycotic activity (M.I.C. expressed in mcg/ml) of RG 024 against myceti, in comparison with miconazole.

| Myceti | RG 024 | Miconazole |
|---|---|---|
| Candida albicans 1 | 1.56 | 3.12 |
| Candida albicans 2 | 0.39 | 1.56 |
| Candida albicans 3 | 0.78 | 1.56 |
| Aspergillus fumigatus 1 | 6.25 | 6.25 |
| Aspergillus fumigatus 2 | 3.12 | 6.25 |
| Microsporum canis 1 | 12.5 | 12.5 |
| Microsporum canis 2 | 6.12 | 6.12 |
| Tricophyton mentagrophites 1 | 0.78 | 1.56 |
| Epidermophyton floccosum 1 | 1.56 | 1.56 |

*"In vitro" antibacterial activity*

"In vitro" antibacterial activity of RG 024 (M.I.C. expressed in mcg/ml) was evaluated in agar culture medium (Isosensitest agar OXOID) on gram-positive and gram-negative microorganisms, both from Standard Collections and hospital isolation wards.

The method was that of scalar dilutions of the test products on agar plates, in a 1:2 dilution ratio. Bacterial suspensions grown overnight in Brain Heart Infusion DIFCO, were diluted 1/100 in new culture medium and inoculated by means of a multiinoculation device.

M.I.C. values were determined by visual observations of bacterial growth, after 18 hours incubation, at 37°C.

The results are shown in Table 2.

TABLE 2
"In vitro" activity (M.I.C. expressed in mcg/ml) of RG 024 on Gram+ and Gram− microorganisms.

| Microorganisms | RG 024 | Miconazole |
|---|---|---|
| Staphylococcus aureus ATCC 9144 | 0.19 | 0.39 |
| Staphylococcus aureus 35 | 0.39 | 6.25 |
| Staphylococcus aureus 38 | 0.19 | 0.39 |
| Streptococcus pyogenes A/1 | 0.19 | 0.39 |
| Streptococcus pyogenes C | 0.39 | 0.39 |
| Streptococcus faecalis OMI 1 | 6.25 | 12.5 |
| Streptococcus faecalis OMI 2 | 6.25 | 6.25 |
| Sarcina lutea ATCC 9341 | 0.19 | 0.19 |
| Escherichia coli OMI 17 | 6.25 | 12.5 |
| Escherichia coli OMI 16 | 6.25 | 25 |
| Klebsiella pneumoniae ATCC 1003 | 12.5 | 25 |
| Proteus vulgaris OMI 4 | 50 | 100 |
| Pseudomonas aeruginosa OMI 1 | >200 | >200 |

The above results clearly show that the values of M.I.C., expressed in mcg/ml, obtained from compound RG 024 are more favourable or equal to those obtained from miconazole, in all of the used microorganisms. Therefore, RG 024 proved to exert remarkable antimycotic and antibacterial activities, higher than those of miconazole.

*PHARMOKINETICS*

The pharmokinetics of RG 024 was evaluated in the rat by oral administration. The serum and urinary concentrations were determined by means of the method of agar-well diffusion in agar culture medium (Sabouraud dextrose agar Difco) seeded with Saccharomyces cerevisiae IS-214 test strain.

The values of said concentrations showed for both compounds a quick absorption, which is remarkably higher for RG 024, in fact the values of the main peak (which for both compounds is recorded about in the first how) are substantially higher (more than 40%) in the case of RG 024.

Moreover, RG 024 is still present in the $8^{th}$ hour, while miconazole may not be detected in significant amounts after the $6^{th}$ hour.

Said trend is proved by the AUC (area under curve) values, which in the case of RG 024 are more than 2.5 times higher than those of miconazole.

The present invention also relates to pharmaceutical compositions containing therapeutically effective amounts of compound I as the active principle.

Said compositions may be administered orally, in form of solid, semi-solid or liquid compositions, such as capsules, tablets, dragees, syrups, oral gels; or topically, in form of powders, lotions, ointments, creams, dermatologic milks or tinctures; vaginal tablets, suppositories or ointments; medicated soaps and the like.

Said pharmaceutical compositions may be of course formulated with conventional excipients, flavouring agents, vehicles, etc.

The pharmaceutical compositions of the invention are used for the treatment of pathological conditions caused by pure or mixed mycotic cutaneous infections, primary and secondary bacterial cutaneous infections, in the treatment and prophylaxys of candidiasis and of oropharyngeus, gastrointestinal and vulvo-vaginal infections.

**Claims for the Contracting States: BE CH DE FR GB IT LI LU NL SE**

1. 1-{[2-(2,4-Dichlorophenyl)-2-(2,4-dichlorophenyl)methoxy]ethyl}-1-pivaloyloxymethyl-1H-imidazolium chloride, having formula I

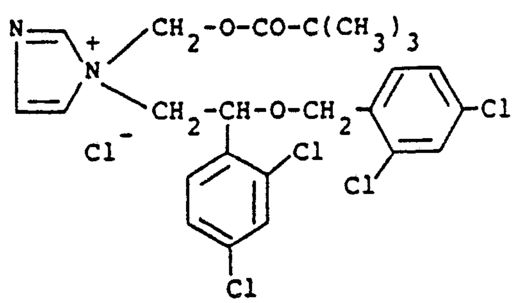

(I)

2. A process for preparing compound of claim 1, wherein 1-(pivaloyloxymethyl)imidazole is reacted with 1-chloro-2-(2,4-dichlorophenyl)-2-[(2,4-dichlorobenzyl)oxy]ethane.

3. A process for preparing compound of claim 1, wherein 1-[2,4-dichloro-β-[(2,4-dichlorobenzyl)oxy]phenetyl]imidazole is reacted with pivalic acid chloromethylester.

4. A process according to claims 2 and 3, wherein the reaction is carried out in almost stoichiometric ratios.

5. Pharmaceutical compositions having antimycotic and antibacterial activities, containing as the active principle compound I together with pharmaceutically acceptable vehicles.

6. Pharmaceutical compositions according to claim 5, in solid, semi-solid or liquid form, for oral or topical administration, for the treatment of humans and/or animals.

**Claims for the Contracting State: AT**

1. Process for preparing 1-{[2-(2,4-dichlorophenyl)-2-(2,4-dichlorophenyl)methoxy]ethyl}-1-pivaloyloxymethyl-1H-imidazolium chloride, having formula I

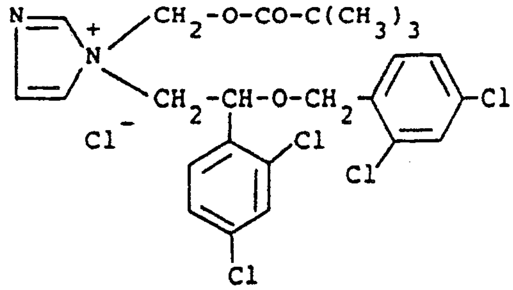

(I)

wherein 1-(pivaloyloxymethyl)imidazole is reacted with 1-chloro-2-(2,4-dichlorophenyl)-2-[(2,4-dichlorobenzyl)oxy]ethane.

2. A process for preparing 1-{[2-(2,4-dichlorophenyl)-2-(2,4-dichlorophenyl)methoxy]ethyl}-1-pivaloyloxymethyl-1H-imidazolium chloride, having formula I

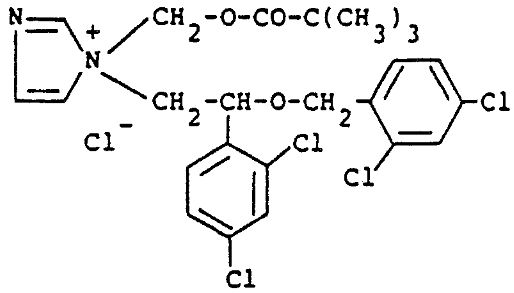

(I)

wherein 1-[2,4-dichloro-β-(2,4-dichlorobenzyl)oxy]phenetyl]imidazole is reacted with pivalic acid chloromethylester.

3. A process according to claims 1 and 2, wherein the reaction is carried out in almost stoichiometric ratios.

**Patentansprüche für die Vertragsstaaten: BE CH DE FR GB IT LI LU NL SE**

1. 1-{[2-(2,4-dichlorphenyl)-2-(2,4-dichlorphenyl)methoxy]ethyl}-1-pivaloyloxymethyl-1H-imidazolium-chlorid mit der Formel I

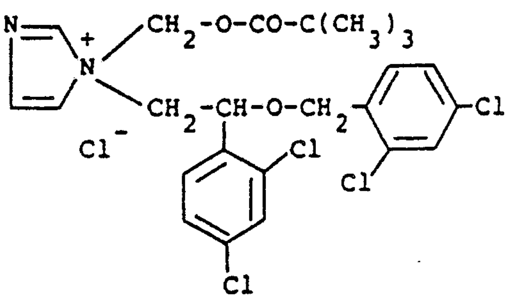

(I)

2. Verfahren zur Herstellung der Verbindung nach Anspruch 1, durch Reaktion von 1-(Pivaloyloxymethyl)imidazol mit 1-Chlor-2-(2,4-dichlorphenyl)-2-[2,4-dichlorbenzyl)oxy]ethan.

3. Verfahren zur Herstellung der Verbindung nach Anspruch 1, durch Reaktion von 1-[2,4-Dichlor-β-[(2,4-dichlorbenzyl)oxy]phenetyl]-imidazol mit Pivalinsäurechlormethylester.

4. Verfahren nach Anspruch 2 und 3, bei dem die Reaktion in fast stoechiometrischen Verhältnissen durchgeführt wird.

5. Pharmazeutische Zusammensetzungen mit antimykotischer und antibakterieller Wirkung, enthaltend als aktives Prinzip die Verbindung I zusammen mit pharmazeutisch brauchbaren Vehikeln.

6. Pharmazeutische Zusammensetzungen nach Anspruch 5 in fester, halbfester oder flüssiger Form zur oralen oder topischen Verabreichung, zur Behandlung von Menschen und/oder Tieren.

**Patentansprüche für den Vertragsstaat: AT**

1. Verfahren zur Herstellung von 1-{[2-(2,4-dichlorphenyl)-2-(2,4-dichlorophenyl)methoxy]ethyl}-1-pivaloyloxymethyl-1H-imidazolium-chlorid mit der Formel I

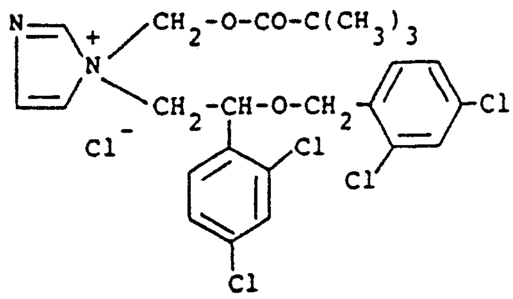

(I)

bei dem 1-(Pivaloyloxymethyl)imidazol mit 1-Chlor-2-(2,4-dichlorphenyl)-2-[(2,4-dichlorbenzyl)-oxy]ethan umgesetzt wird.

2. Verfahren zur Herstellung von 1-{[2-(2,4-Dichlorphenyl)-2-(2,4-dichlorphenyl)methoxy]ethyl}-1-pivaloyloxymethyl-1H-imidazolium-chlorid mit der Formel I

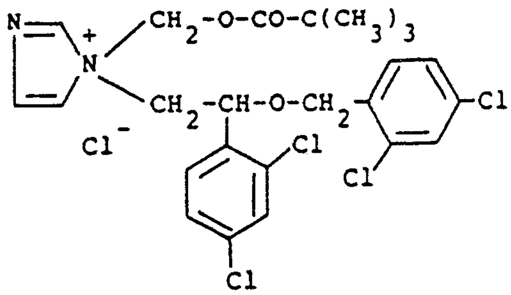

(I)

bei dem 1-[2,4-Dichlor-β-[(2,4-dichlorbenzyl)oxy]phenetyl]imidazol mit Pivalinsäure-chlormethylester umgesetzt wird.

3. Verfahren nach den Ansprüchen 1 und 2, bei dem die Reaktion in fast stoechiometrischen Verhältnissen durchgeführt wird.

**Revendications pour les Etats contractants: BE CH DE FR GB IT LI LU NL SE**

1. Chlorure de 1-{[2-(2,4-dichlorophényl)-2-(2,4-dichlorophényl)methoxy]éthyl}-1-pivaloyloxyméthyl-1H-imidazolium de formule I

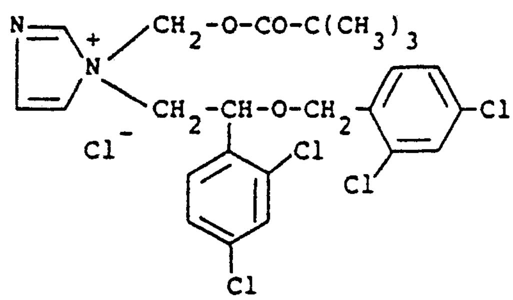

(I)

2. Procédé de préparation du composé selon la revendication 1, qui consiste à faire réagire le 1-(pivaloyloxyméthyl)imidazole avece le 1-chloro-2-(2,4-dichlorophényl)-2-[(2,4-dichlorobenzyl)oxy]éthane.

3. Procédé de préparation du composé selon la revendication 1, qui consiste à faire réagir le 1-[2,4-dichloro-β-[(2,4-dichlorobenzyl)oxy]phénétyl]-imidazole avec l'ester chlorométhylique de l'acide pivalique.

4. Procédé selon la revendication 2 ou 3, dans laquelle la réaction est effectuée avec des rapports pratiquement stoechiométriques.

5. Compositions pharmaceutiques ayant des activités antimycotiques et antibactériennes, contenant à titre de principe actif le composé I avec des véhicules pharmaceutiquement acceptables.

6. Compositions pharmaceutiques selon la revendication 5, sous forme solide, semi-solide ou liquide, pour une administration orale ou topique, pour le traitement des humains et/ou des animaux.

**Revendications pour l'Etat contractant: AT**

1. Procédé de préparation du chlorure de 1-{[2-(2,4-dichlorophényl)-2-(2,4-dichlorophényl) methoxy]éthyl}-1-pivaloyloxyméthyl-1H-imidazolium de formule I

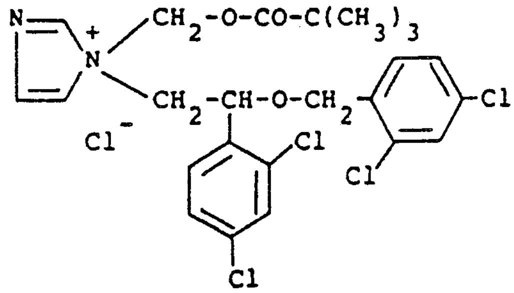

(I)

qui consiste à faire réagir le 1-(pivaloyloxyméthyl)imidazole avec le 1-chloro-2-(2,4-dichlorophényl)-2-[(2,4-dichlorobenzyl)oxy]éthane.

2. Procédé de préparation du chlorure de 1-{[2-(2,4-dichlorophényl)-2-(2,4-dichlorophényl)méthoxy)-éthyl}-1-pivaloyloxyméthyl-1H-imidazolium de formule I

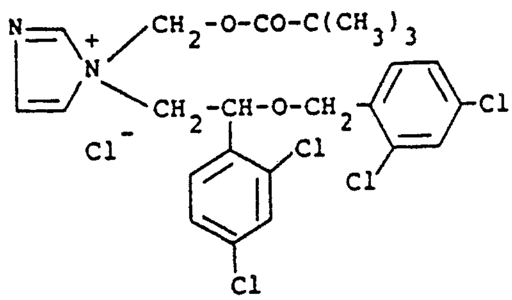

(I)

qui consiste à faire réagir le 1-[2,4-dichloro-β-[(2,4-dichlorobenzyl)oxy]phénétyl]-imidazole avec l'ester chlorométhylique de l'acide pivalique.

3. Procédé selon la revendication 1 ou 2, dans laquelle la réaction est effectuée avec des rapports pratiquement stoechiométriques.